# EUROPEAN PATENT APPLICATION

(11) **EP 3 301 096 A1**
(43) Date of publication of application: **04.04.2018**
(21) Application number: 16191804.0
(22) Date of filing: 30.09.2016
(51) Int. Cl.: C07D 487/02, A61K 31/40, A61P 31/04

(54) **BIS-INDOLE DERIVATIVES FOR USE IN THE TREATMENT OF BACTERIAL INFECTIONS**

(71) Applicant: Heinrich Heine Universität Düsseldorf, 40225 Düsseldorf (DE)
(72) Inventor: Müller, Thomas J. J., 40591 Düsseldorf (DE); Sommer, Gereon, 40227 Düsseldorf (DE); Tasch, Boris O. A., 40589 Düsseldorf (DE); Kalscheuer, Rainer, 40724 Hilden (DE); Rehberg, Nidja, 40545 Düsseldorf (DE)
(74) Representative: Michalski Hüttermann & Partner Patentanwälte mbB

(57) **Abstract**

The present invention relates to compounds according to general formula (1) for use in the treatment of a bacterial infection, particularly for use in the treatment of a bacterial infection with methicillin resistant *Staphylococcus aureus* (MRSA), and a pharmaceutical composition comprising compounds according to general formula (1) for use in the treatment of a bacterial infection.

## Description

The present invention relates to compounds for use in the treatment of bacterial infections.

Since the introduction of penicillin, antibiotics have become one of the cornerstones of modem medicine. The broad use of antibiotics in recent years however has significantly increased the number of resistant microorganisms, which are more and more associated with an increased risk of mortality among hospitalized patients becoming one of the most significant problems within the healthcare industry. Among the resistant microorganisms, methicillin-resistant *Staphylococcus aureus* (MRSA) represents an increasingly problematic microorganism. Methicillin-resistant *S. aureus* not only is resistant to currently available β-lactam antibiotics such as penicillin, but generally to most antibiotics of frequent therapeutical use. Strains of methicillin-resistant *S. aureus* have become increasingly common and the spread of these represents a serious health threat. An infection with MRSA increases the risk of dying during hospitalization in Germany by a factor of 2.7. The speed and the frequency of the occurrence of reduced susceptibility to antibiotics generally is considered alarming.

In the treatment of infections with MRSA some antibiotics such as vancomycin, lenozolid and tigecyclin still are useful and combination antibiotic treatment is widely practiced in the clinic. These antibiotics however are expensive, and vancomycin-resistant *S. aureus* strains already have developed which reduces clinical applicability. On the one hand, commercial development of new classes of antibiotics has diminished over the past years. The search for new antibacterial compounds thus is a constant challenge in pharmaceutical research.

Therefore, the object underlying the present invention was to provide compounds which provide antibacterial activity, and particularly provide inhibitory activity against MRSA.

The problem is solved by a compound according to general formula (1) as given as follows and/or racemates, enantiomers, stereoisomers, solvates, hydrates, and pharmaceutically acceptable salts and/or esters thereof: 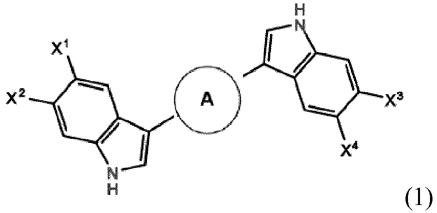 wherein:
- A: is a 6-membered heteroaromatic ring selected from the group consisting of pyridyl, pyridazyl, pyrimidyl and/or pyrazyl, or a 5-membered heteroaromatic ring selected from the group consisting of thiazolyl, oxazolyl, imidazolyl, pyrazolyl, thiophenyl, furyl and/or pyrrolyl; and
- X¹, X⁴: are selected from Cl or Br and X², X³ are hydrogen if A is a 6-membered heteroaromatic ring, or
- X¹, X², X³, X⁴: are the same or independent from each other selected from the group consisting of H, Cl and Br if A is a 5-membered heteroaromatic ring,
for use in the treatment of a bacterial infection.

Surprisingly, it was found that the compounds showed inhibitory activity against Gram-positive bacterial strains. Particularly, the compounds showed inhibitory activity against methicillin resistant *Staphylococcus aureus* (MRSA). Beneficially, the compounds thus are useful in the treatment of a bacterial infection, particularly an infection with MRSA.

In embodiments, the linker A is a 6-membered heteroaromatic ring selected from the group consisting of pyridyl, pyridazyl, pyrimidyl and/or pyrazyl, the substituents X¹, X⁴ are selected from Cl or Br, and and the substituents X², X³ are hydrogen. It is assumed that a halogenation with chlorine or bromine at position 5, 5' of the indole ring is advantageous for the antimicrobial activity of the compounds comprising a 6-membered heteroaromatic ring. Preferably, a 6-membered heteroaromatic ring is substituted with the indole rings in para-position. Preferably, the linker A is a 6-membered heteroaromatic ring selected from pyrimidyl and pyrazyl. These compounds represent alocasin A and hyrtinadine A derivatives. In preferred embodiments, A is pyrimidyl or pyrazyl, X¹, X⁴ are selected from Cl or Br, and X², X³ are hydrogen. It was found that these alocasin A and hyrtinadine A derivatives showed high inhibitory activity against methicillin resistant *Staphylococcus aureus* (MRSA). In especially preferred embodiments, the compound is selected from the group of compounds according to formulas (2) to (4) as given as follows:

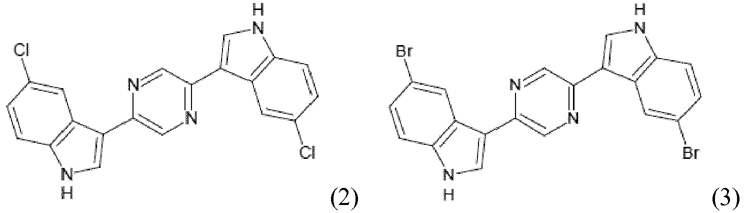

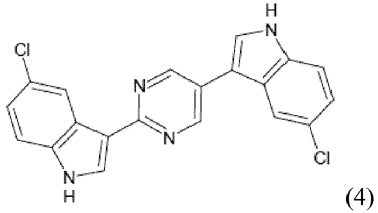

Advantageously, the compounds according to formulas (2), (3) and (4) showed particularly strong bactericidal effect against MRSA. Further, the compounds of formulas (2), (3) and (4) only were cytotoxic at sufficiently high concentration, so as to provide favorable selectivity indices and thus yielding sufficient therapeutic margins. The hyrtinadine A derivative of formula (4) advantageously showed good bactericidal effects also against other Gram-positive bacteria, such as strains of *Staphylococcus aureus,* particularly with methicillin resistant *Staphylococcus aureus* (MRSA), *Enterococcus faecalis* and *Enterococcus faecium.*

In further embodiments, the linker A is a 5-membered heteroaromatic ring selected from the group consisting of thiazolyl, oxazolyl, imidazolyl, pyrazolyl, thiophenyl, furyl and/or pyrrolyl and the substituents X¹, X², X³, X⁴ are the same or independent from each other selected from the group consisting of hydrogen, Cl and Br. 5-membered heteroaromatic rings maybe substituted with indole rings in 3,5- or 3,4-position. Preferrably, 5-membered heteroaromatic rings, particularly thiazolyl rings, are substituted with indole rings in 3,5-position. It was found that compounds comprising a 5-membered heteroaromatic ring also showed good antibacterial effects if the substituents are hydrogen. In a preferred embodiment, A is thiazolyl and X¹, X², X³ and X⁴ are hydrogen. It was found that the thiazolyl compound showed high inhibitory activity against *Staphylococcus aureus,* particularly methicillin resistant *Staphylococcus aureus* (MRSA) and *Enterococcus faecium.* In especially preferred embodiments, the compound comprising a 5-membered heteroaromatic ring is selected from the group of compounds according to formulas (18) to (21) as given as follows:

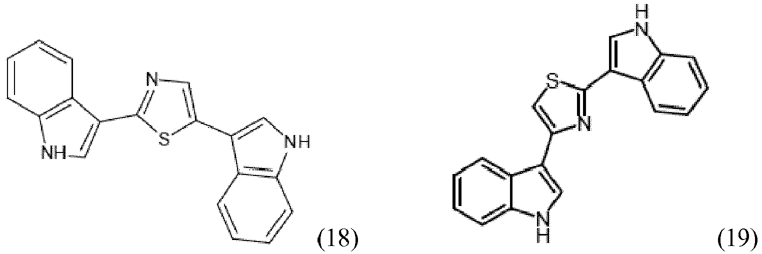

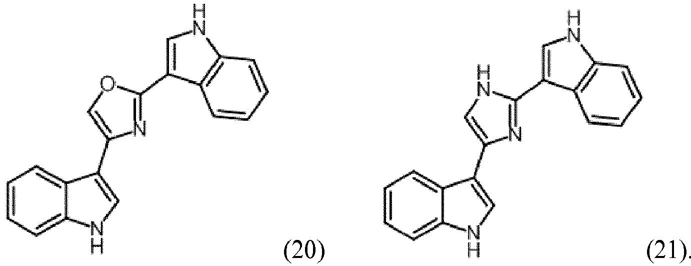

The compounds are usable against Gram-positive bacterial strains such as strains of *Staphylococcus aureus, Enterococcus faecalis* and *Enterococcus faecium.* In preferred embodiments, the compounds are useable in the treatment of a bacterial infection with *Staphylococcus aureus,* particularly with methicillin resistant *Staphylococcus aureus* (MRSA). A particular advantage is that the compounds show bactericidal activity especially against the methicillin resistant *Staphylococcus aureus* strain.

The compounds for use in the treatment of a bacterial infection may be formulated as a pharmaceutical composition. A further aspect of the present invention relates to a pharmaceutical composition comprising as an active ingredient at least one compound according to general formula (1) and/or racemates, enantiomers, stereoisomers, solvates, hydrates, and pharmaceutically acceptable salts and/or esters thereof: 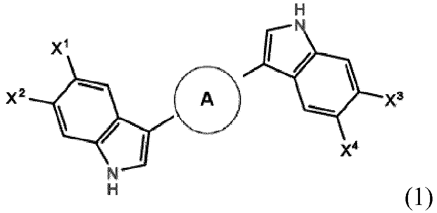 wherein:
- A: is a 6-membered heteroaromatic ring selected from the group consisting of pyridyl, pyridazyl, pyrimidyl and/or pyrazyl, or a 5-membered heteroaromatic ring selected from the group consisting of thiazolyl, oxazolyl, imidazolyl, pyrazolyl, thiophenyl, furyl and/or pyrrolyl; and
- X¹, X⁴: are selected from Cl or Br and X², X³ are hydrogen if A is a 6-membered heteroaromatic ring, or
- X¹, X², X³, X⁴: are the same or independent from each other selected from the group consisting of H, Cl and Br if A is a 5-membered heteroaromatic ring,
for use in the treatment of a bacterial infection.

In embodiments, the linker A is a 6-membered heteroaromatic ring selected from the group consisting of pyridyl, pyridazyl, pyrimidyl and/or pyrazyl, the substituents X¹, X⁴ are selected from Cl or Br, and and the substituents X², X³ are hydrogen. Preferably, a 6-membered heteroaromatic ring is substituted with the indole rings in para-position. Preferably, the linker A is a 6-membered heteroaromatic ring selected from pyrimidyl and pyrazyl. In preferred embodiments, A is pyrimidyl or pyrazyl, X¹, X⁴ are selected from Cl or Br, and X², X³ are hydrogen. In preferred embodiments, the pharmaceutical composition comprises as an active ingredient an alocasin A or hyrtinadine A derivative selected from the compounds according to formulas (2) to (4). In further embodiments, the linker A is a 5-membered heteroaromatic ring selected from the group consisting of thiazolyl, oxazolyl, imidazolyl, pyrazolyl, thiophenyl, furyl and/or pyrrolyl and the substituents X¹, X², X³, X⁴ are the same or independent from each other selected from the group consisting of hydrogen, Cl and Br. 5-membered heteroaromatic rings may be substituted with indole rings in 3,5- or 3,4-position. Preferrably, 5-membered heteroaromatic rings, particularly thiazolyl rings, are substituted with indole rings in 3,5-position. In a preferred embodiment, A is thiazolyl and X¹, X², X³ and X⁴ are hydrogen. In preferred embodiments, the pharmaceutical composition comprises as an active ingredient a compounds selected from the compounds according to formulas (18) to (21).

The pharmaceutical composition may comprise as an active ingredient at least one compound according to general formula (1). In embodiments, the pharmaceutical composition may comprise a mixture of two or more of the compounds according to general formula (1). For example the compositiom may comprise a mixture of the compounds according to formulas (2) to (4) and (18) to (21). A useful combination may depend on the bacterial infection to be treated. In further embodiments, the compounds and/or racemates, enantiomers, diastereomers, solvates, hydrates, and pharmaceutically acceptable salts and/or esters thereof can be included in a pharmaceutical composition in combination with one or more other therapeutically active compounds, particularly in combination with further antibiotic substances.

The pharmaceutical composition is usable in the treatment of a bacterial infection with Gram-positive bacterial strains such as strains of *Staphylococcus aureus, Enterococcus faecalis* and *Enterococcus faecium.* In preferred embodiments, the pharmaceutical composition is useable in the treatment of a bacterial infection with *Staphylococcus aureus*, particularly with methicillin resistant *Staphylococcus aureus* (MRSA).

The compounds particularly are usable in form of solvates, hydrates, and pharmaceutically acceptable salts and/or esters thereof. The term "pharmaceutically acceptable salts" refers to salts prepared from pharmaceutically acceptable non-toxic bases or acids. A pharmaceutically acceptable salt can be conveniently prepared from pharmaceutically acceptable non-toxic bases, including inorganic bases and organic bases, organic anions, organic cations, halides or alkaline. The term pharmaceutically acceptable salt includes alkali metal salts and addition salts of free acids or free bases. Suitable pharmaceutically acceptable base addition salts include metallic salts and organic salts. Preferred salts derived from inorganic bases include ammonium, calcium, magnesium, potassium and sodium salts. Salts derived from pharmaceutically acceptable organic non-toxic bases include salts of primary, secondary, and tertiary amines. The compounds may be used in the form of a hydrochloride or maleate.

The pharmaceutical composition can comprise a compound according to general formula (1) as an active ingredient, a pharmaceutically acceptable carrier and optionally other therapeutic ingredients or adjuvants. The present invention hence also relates to a pharmaceutical composition for use in the treatment of a bacterial infection wherein the composition comprises as an active ingredient a compound according to general formula (1) and a pharmaceutically acceptable carrier. The compound can be dissolved or dispersed in a pharmaceutically acceptable carrier. The term "pharmaceutical or pharmacologically acceptable" refers to molecular entities and compositions that do not produce an adverse, allergic or other untoward reaction when administered to a subject, such as, for example, a human, as appropriate. The pharmaceutical carrier can be, for example, a solid, liquid, or gas.

Suitable carriers and adjuvants can correspond to substances ordinarily employed in formulation technology for pharmaceutical formulations. Examples of solid carriers include lactose, terra alba, sucrose, talc, gelatin, agar, pectin, acacia, magnesium stearate, and stearic acid. For example, water, glycols, oils, alcohols and the like may be used as liquid carriers to form liquid preparations such as solutions. Preferred examples of liquid carriers are sugar syrup, peanut oil, olive oil, and water. In preferred embodiments, the pharmaceutical composition comprises a liquid or solid carrier.

In embodiments, the compounds may be dissolved in a suitable solvent, for example dimethylsulfoxide. For example, the compounds may be dissolved in dimethylsulfoxide and provided as a suspension in an aqueous liquid, preferably using water as liquid carrier. A solution may comprise in a range of 0.02 % to 0.08 % (v/v) dimethylsulfoxide.

The compositions can be suitable for oral, dermal, rectal, topical, and parenteral administration. The pharmaceutical compositions may be conveniently presented in unit dosage form and prepared by any of the methods well known in the art of pharmacy. The pharmaceutical composition of the present invention can be presented as discrete unit suitable for oral administration such as capsules, cachets or tablets each containing a predetermined amount of the active ingredient. Further, the compositions can be presented as a powder, as granules, as a solution, as a suspension in an aqueous liquid, in a non-aqueous liquid, as an oil-in-water emulsion or as a water-in-oil liquid emulsion. Further, the pharmaceutical composition may be administered by controlled release means and/or delivery devices.

The present invention also relates to the use of a compound according to general formula (1) and/or racemates, enantiomers, stereoisomers, solvates, hydrates, and pharmaceutically acceptable salts and/or esters thereof: 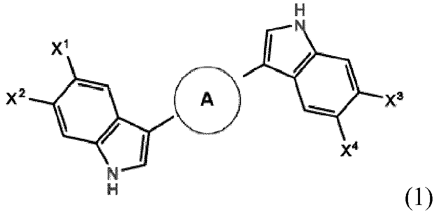 wherein:
- A: is a 6-membered heteroaromatic ring selected from the group consisting of pyridyl, pyridazyl, pyrimidyl and/or pyrazyl, or a 5-membered heteroaromatic ring selected from the group consisting of thiazolyl, oxazolyl, imidazolyl, pyrazolyl, thiophenyl, furyl and/or pyrrolyl; and
- X¹, X⁴: are selected from Cl or Br and X², X³ are hydrogen if A is a 6-membered heteroaromatic ring, or
- X¹, X², X³, X⁴: are the same or independent from each other selected from the group consisting of H, Cl and Br if A is a 5-membered heteroaromatic ring,
for the manufacture of a medicament for the treatment of a bacterial infection.

The use of the compounds can relate to a treatment of a bacterial infection with Gram-positive bacterial strains such as strains of *Staphylococcus aureus, Enterococcus faecalis* and *Enterococcus faecium.* In preferred embodiments, the compounds are usable for the manufacture of a medicament for the treatment of a bacterial infection with *Staphylococcus aureus,* particularly with methicillin resistant *Staphylococcus aureus* (MRSA). In embodiments, the linker A is a 6-membered heteroaromatic ring selected from the group consisting of pyridyl, pyridazyl, pyrimidyl and/or pyrazyl, the substituents X¹, X⁴ are selected from Cl or Br, and the substituents X², X³ are hydrogen. Preferably, a 6-membered heteroaromatic ring is substituted with the indole rings in para-position. Preferably, the linker A is a 6-membered heteroaromatic ring selected from pyrimidyl and pyrazyl. In preferred embodiments, A is pyrimidyl or pyrazyl, X¹, X⁴ are selected from Cl or Br, and X², X³ are hydrogen. In preferred embodiments, the pharmaceutical composition comprises as an active ingredient an alocasin A or hyrtinadine A derivative selected from the compounds according to formulas (2) to (4). In further embodiments, the linker A is a 5-membered heteroaromatic ring selected from the group consisting of thiazolyl, oxazolyl, imidazolyl, pyrazolyl, thiophenyl, furyl and/or pyrrolyl and the substituents X¹, X², X³, X⁴ are the same or independent from each other selected from the group consisting of hydrogen, Cl and Br. 5-membered heteroaromatic rings may be substituted with indole rings in 3,5- or 3,4-position. Preferrably, 5-membered heteroaromatic rings, particularly thiazolyl rings, are substituted with indole rings in 3,5-position. In a preferred embodiment, A is thiazolyl and X¹, X², X³ and X⁴ are hydrogen. In preferred embodiments, the pharmaceutical composition comprises as an active ingredient a compounds selected from the compounds according to formulas (18) to (21).

A further aspect of the present invention relates to a method of treating a bacterial infection, the method comprising the step of administering to a subject a therapeutically effective amount of a compound according to general formula (1) and/or racemates, enantiomers, stereoisomers, solvates, hydrates, and pharmaceutically acceptable salts and/or esters thereof: 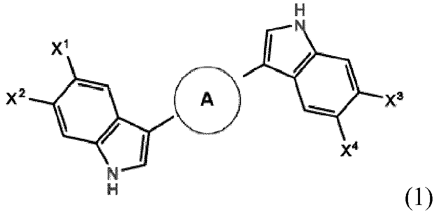 wherein:
- A: is a 6-membered heteroaromatic ring selected from the group consisting of pyridyl, pyridazyl, pyrimidyl and/or pyrazyl, or a 5-membered heteroaromatic ring selected from the group consisting of thiazolyl, oxazolyl, imidazolyl, pyrazolyl, thiophenyl, furyl and/or pyrrolyl; and
- X¹, X⁴: are selected from Cl or Br and X², X³ are hydrogen if A is a 6-membered heteroaromatic ring, or
- X¹, X², X³, X⁴: are the same or independent from each other selected from the group consisting of H, Cl and Br if A is a 5-membered heteroaromatic ring.

The method of treating a bacterial infection may relate to a bacterial infection with Gram-positive bacterial strains such as strains of *Staphylococcus aureus, Enterococcus faecalis* and *Enterococcus faecium.* In embodiments, the bacterial infection is an infection with *Staphylococcus aureus,* particularly with methicillin resistant *Staphylococcus aureus* (MRSA).

Subjects include both human subjects and animal subjects, particularly mammalian subjects such as human subjects or mice or rats for medical purposes. The term "therapeutically effective amount" is used herein to mean an amount or dose sufficient to cause an improvement in a clinically significant condition in the subject. The method may include oral or parenteral administration.

In embodiments, the linker A is a 6-membered heteroaromatic ring selected from the group consisting of pyridyl, pyridazyl, pyrimidyl and/or pyrazyl, the substituents X¹, X⁴ are selected from Cl or Br, and and the substituents X², X³ are hydrogen. Preferably, a 6-membered heteroaromatic ring is substituted with the indole rings in para-position. Preferably, the linker A is a 6-membered heteroaromatic ring selected from pyrimidyl and pyrazyl. In preferred embodiments, A is pyrimidyl or pyrazyl, X¹, X⁴ are selected from Cl or Br, and X², X³ are hydrogen. In preferred embodiments, the pharmaceutical composition comprises as an active ingredient an alocasin A or hyrtinadine A derivative selected from the compounds according to formulas (2) to (4). In further embodiments, the linker A is a 5-membered heteroaromatic ring selected from the group consisting of thiazolyl, oxazolyl, imidazolyl, pyrazolyl, thiophenyl, furyl and/or pyrrolyl and the substituents X¹, X², X³, X⁴ are the same or independent from each other selected from the group consisting of hydrogen, Cl and Br. 5-membered heteroaromatic rings may be substituted with indole rings in 3,5- or 3,4-position. Preferrably, 5-membered heteroaromatic rings, particularly thiazolyl rings, are substituted with indole rings in 3,5-position. In a preferred embodiment, A is thiazolyl and X¹, X², X³ and X⁴ are hydrogen. In preferred embodiments, the pharmaceutical composition comprises as an active ingredient a compounds selected from the compounds according to formulas (18) to (21).

A further aspect of the present invention relates to a compound according to general formula (1) as given as follows and/or racemates, enantiomers, stereoisomers, solvates, hydrates, and pharmaceutically acceptable salts and/or esters thereof: 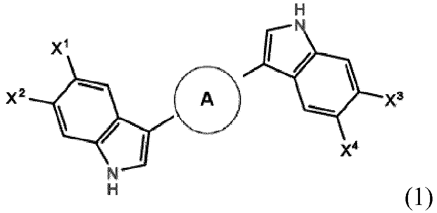 wherein:
- A: is a 6-membered heteroaromatic ring selected from the group consisting of pyridyl, pyridazyl, pyrimidyl and/or pyrazyl, or a 5-membered heteroaromatic ring selected from the group consisting of thiazolyl, oxazolyl, imidazolyl, pyrazolyl, thiophenyl, furyl and/or pyrrolyl; and
- X¹ X², X³, X⁴: are the same or independent from each other selected from the group consisting of H, OCH₃, Cl, F and Br.

In embodiments, A is a 6-membered heteroaromatic ring selected from the group consisting of pyridyl, pyridazyl, pyrimidyl and/or pyrazyl, and X¹, X⁴ are selected from Cl or Br and X², X³ are hydrogen. Preferably, a 6-membered heteroaromatic ring is substituted with the indole rings in para-position. Preferably, the linker A is a 6-membered heteroaromatic ring selected from pyrimidyl and pyrazyl. In preferred embodiments, the compound is an alocasin A derivative selected from the compounds according to formulas (19) to (21) or the compounds according to formulas (2), (5), (7) and (9) as given as follows:

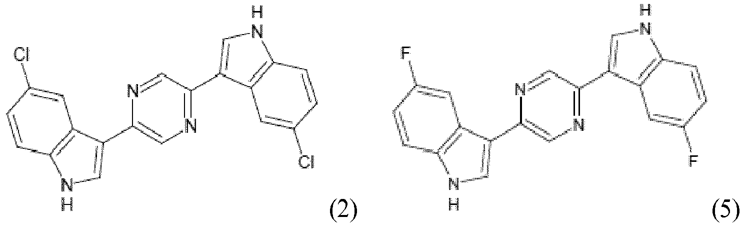

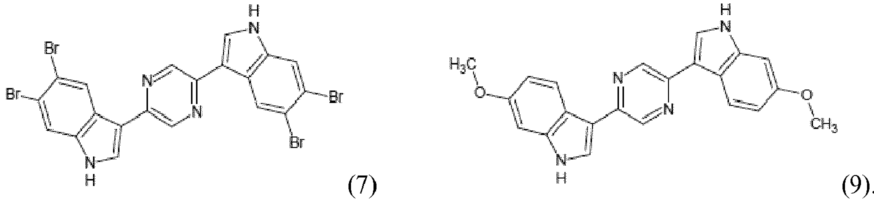

In most preferred embodiments, the compound is the alocasin A derivative according to formula (2).

In further embodiments, the linker A is a 5-membered heteroaromatic ring selected from the group consisting of thiazolyl, oxazolyl, imidazolyl, pyrazolyl, thiophenyl, furyl and/or pyrrolyl and the substituents X¹, X², X³, X⁴ are the same or independent from each other selected from the group consisting of hydrogen, Cl and Br. 5-membered heteroaromatic rings maybe substituted with indole rings in 3,5- or 3,4-position. Preferrably, 5-membered heteroaromatic rings, particularly thiazolyl rings, are substituted with indole rings in 3,5-position. In a preferred embodiment, A is thiazolyl and X¹, X², X³ and X⁴ are hydrogen. In preferred embodiments, the pharmaceutical composition comprises as an active ingredient a compounds selected from the compounds according to formulas (18) to (21).

The compounds may be usable as a medicament, particularly for the treatment of a bacterial infection, or as an inhibitor of the enzyme EIICBA.

A further aspect of the present invention relates to a compound according to general formula (1) as given as follows and/or racemates, enantiomers, stereoisomers, solvates, hydrates, and pharmaceutically acceptable salts and/or esters thereof: 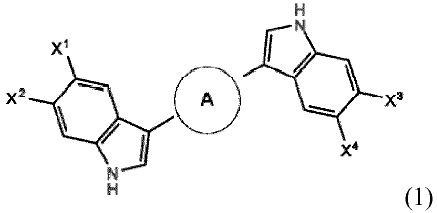 wherein:
- A: is a 6-membered heteroaromatic ring selected from the group consisting of pyridyl, pyridazyl, pyrimidyl and/or pyrazyl, or a 5-membered heteroaromatic ring selected from the group consisting of thiazolyl, oxazolyl, imidazolyl, pyrazolyl, thiophenyl, furyl and/or pyrrolyl; and
- X¹, X⁴: are selected from Cl or Br and X², X³ are hydrogen if A is a 6-membered heteroaromatic ring, or
- X¹, X², X³, X⁴: are the same or independent from each other selected from the group consisting of H, Cl and Br if A is a 5-membered heteroaromatic ring,
for use as a medicament.

In embodiments, the linker A is a 6-membered heteroaromatic ring selected from the group consisting of pyridyl, pyridazyl, pyrimidyl and/or pyrazyl, the substituents X¹, X⁴ are selected from Cl or Br, and and the substituents X², X³ are hydrogen. Preferably, a 6-membered heteroaromatic ring is substituted with the indole rings in para-position. Preferably, the linker A is a 6-membered heteroaromatic ring selected from pyrimidyl and pyrazyl. In preferred embodiments, A is pyrimidyl or pyrazyl, X¹, X⁴ are selected from Cl or Br, and X², X³ are hydrogen. In preferred embodiments, the compound is an alocasin A or hyrtinadine A derivative selected from the compounds according to formulas (2) to (4). In further embodiments, the linker A is a 5-membered heteroaromatic ring selected from the group consisting of thiazolyl, oxazolyl, imidazolyl, pyrazolyl, thiophenyl, furyl and/or pyrrolyl and the substituents X¹, X², X³, X⁴ are the same or independent from each other selected from the group consisting of hydrogen, Cl and Br. 5-membered heteroaromatic rings may be substituted with indole rings in 3,5- or 3,4-position. Preferrably, 5-membered heteroaromatic rings, particularly thiazolyl rings, are substituted with indole rings in 3,5-position. In a preferred embodiment, A is thiazolyl and X¹, X², X³ and X⁴ are hydrogen. In preferred embodiments, the compound is selected from the compounds according to formulas (18) to (21). A preferred aspect relates to the compounds according to formulas (2) and (18) for use as a medicament.

A further aspect of the present invention relates to inhibitors of the enzyme EIICBA, particularly inhibitors according to general formula (1) as given as follows and/or racemates, enantiomers, stereoisomers, solvates, hydrates, and pharmaceutically acceptable salts and/or esters thereof: 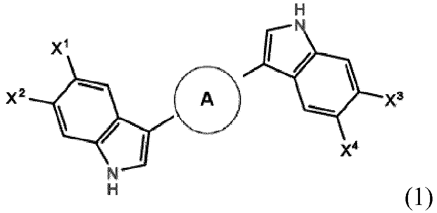 wherein:
- A: is a 6-membered heteroaromatic ring selected from the group consisting of pyridyl, pyridazyl, pyrimidyl and/or pyrazyl, or a 5-membered heteroaromatic ring selected from the group consisting of thiazolyl, oxazolyl, imidazolyl, pyrazolyl, thiophenyl, furyl and/or pyrrolyl; and
- X¹, X⁴: are selected from Cl or Br and X², X³ are hydrogen if A is a 6-membered heteroaromatic ring, or
- X¹, X², X³, X⁴: are the same or independent from each other selected from the group consisting of H, Cl and Br if A is a 5-membered heteroaromatic ring.

The enzyme EIICBA is a component from the phosphoenolpyruvate-dependent glucose phosphotransferase system (PTS). The accepted name of EIICBA is protein-*N*^{π}-phosphohistidine-*N-*acetyl-D-glucosamine phosphotransferase.

In embodiments, the linker A is a 6-membered heteroaromatic ring selected from the group consisting of pyridyl, pyridazyl, pyrimidyl and/or pyrazyl, the substituents X¹, X⁴ are selected from Cl or Br, and and the substituents X², X³ are hydrogen. Preferably, a 6-membered heteroaromatic ring is substituted with the indole rings in para-position. Preferably, the linker A is a 6-membered heteroaromatic ring selected from pyrimidyl and pyrazyl. In preferred embodiments, A is pyrimidyl or pyrazyl, X¹, X⁴ are selected from Cl or Br, and X², X³ are hydrogen. In preferred embodiments, the inhibitor is an alocasin A or hyrtinadine A derivative selected from the compounds according to formulas (2) to (4). In further embodiments, the linker A is a 5-membered heteroaromatic ring selected from the group consisting of thiazolyl, oxazolyl, imidazolyl, pyrazolyl, thiophenyl, furyl and/or pyrrolyl and the substituents X¹ X², X³, X⁴ are the same or independent from each other selected from the group consisting of hydrogen, Cl and Br. 5-membered heteroaromatic rings may be substituted with indole rings in 3,5- or 3,4-position. Preferrably, 5-membered heteroaromatic rings, particularly thiazolyl rings, are substituted with indole rings in 3,5-position. In a preferred embodiment, A is thiazolyl and X¹ X², X³ and X⁴ are hydrogen. In preferred embodiments, the inhibitor is selected from the compounds according to formulas (18) to (21).

Unless otherwise defined, the technical and scientific terms used herein have the same meaning as commonly understood by one of ordinary skill in the art to which this invention belongs.

The examples which follow serve to illustrate the invention in more detail but do not constitute a limitation thereof.

The figures show:

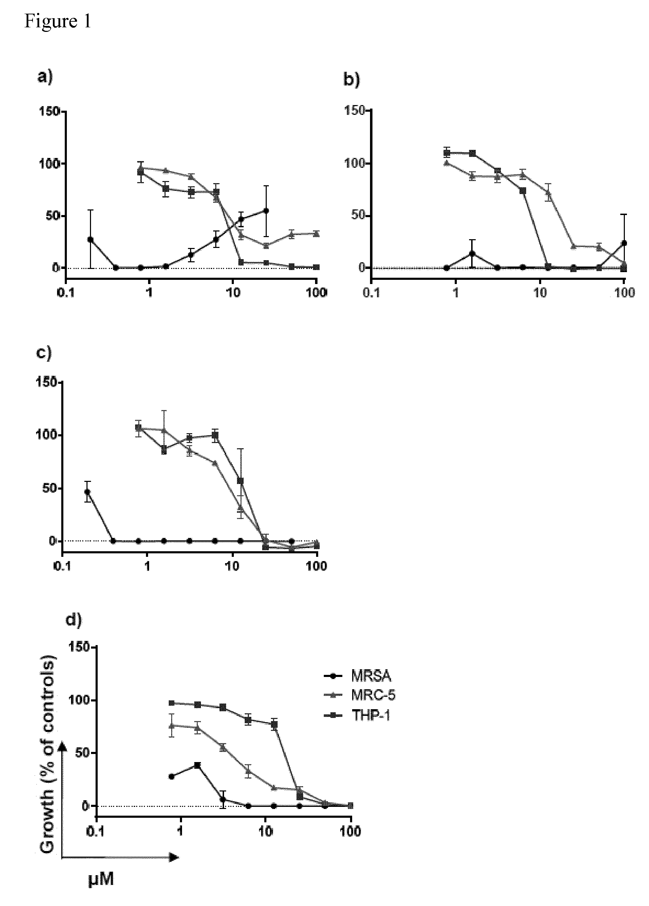

- Figure 1: Dose-response curves of the compounds according to formula (2) in Figure 1a), formula (3) in Figure 1b), formula (4) in Figure 1c), and (18) in Figure 1d) against MRSA (●) and the human cell lines MRC-5 (▲) and THP-1 (■);

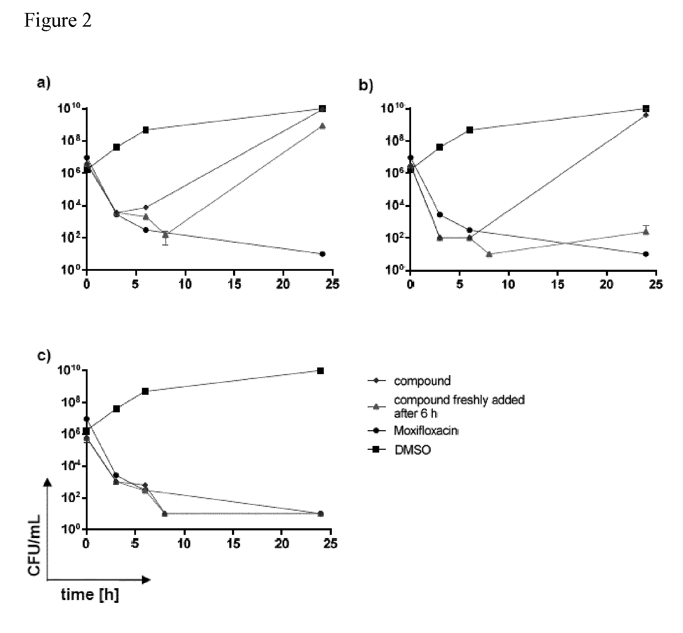

- Figure 2: Time-kill curves of methicillin resistant *S. aureus* for compounds according to formula (2) in Figure 2a), formula (3) in Figure 2b), and formula (4) in Figure 2c). Compounds were tested at 5x MIC resulting in 0.02-0.08% DMSO final concentration (◆). Additionally, in an independent experiment, medium was replaced and compounds were freshly added after 6 h (A). Moxifloxacin at 5x MIC and cells grown in medium without antibiotic but only containing 0.08% DMSO were included as positive and negative controls, respectively. Results are presented as the mean of duplicate experiments ± SD.;

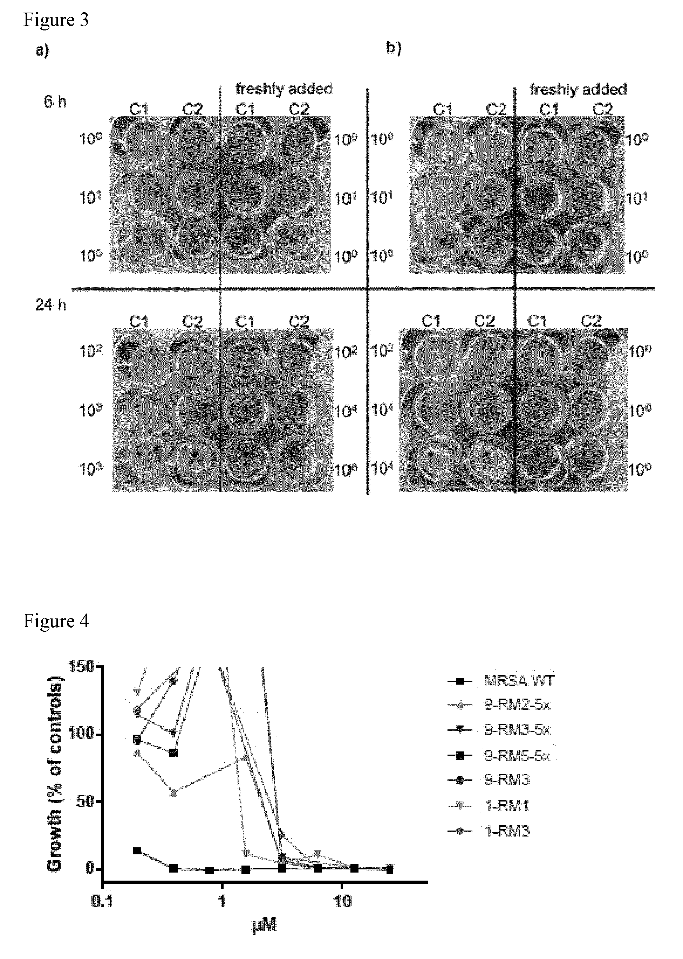

- Figure 3: Resistance development in Methicillin resistant *S. aureus* (MRSA; ATCC 700699) during time-kill kinetics of the compounds according to formula (2) in Figure 3a), and formula (3) in Figure 3b). Two independent cultures C1 and C2 were tested for each attempt. Compounds were tested at 5x MIC at 0.02-0.08% DMSO final concentration. In an independent experiment, medium was replaced and compounds were freshly added after 6 h ("freshly added"). 20 µL of different dilution of the cultures after either 6 or 24 h of incubation were plated out on MH agar containing either no antibiotics (*) or the compounds according to formula (2) in Figure 3a), formula (3) in Figure 3b) at 5x MIC, respectively.;
- Figure 4: Dose-response curves of resistant MRSA mutants for the compound according to formula (4). Mutants were generated on agar containing 3-fold MIC of the compound according to formula (4) (9-RM) or formula (2) (1-RM) or with 5-fold MIC of the compound according to formula (4) (9-RM-5x). Parent MRSA strain (MRSA WT) was used as control.

### Example 1

### Synthesis of the compound according to formula (2): 2,5-bis(5-chloro-1H-indole-3-yl)pyrazine

0.432 g 5-Chloro-3-iodo-1-tosyl-indole (1.0 mmol, 1 eq.) and 0.05 g Pd(PPh₃)₄ (0.05 mmol, 0.05 eq.) were suspended in 5 ml dry 1,4-dioxane in a dry Schlenk tube under nitrogen. 1.4 ml triethylamine (10 mmol, 10 eq.) and 0.23 ml pinacolborane (1.5 mmol, 1.5 eq.) were added dropwise and the reaction mixture was stirred at 80° C for 4 h. Thereafter the mixture was cooled to room temperature (20 ± 2 °C) and methanol (5 mL), 0.82 g cesium carbonate (2.50 mmol, 2.5 eq.) and 0.118 g 2,5-dibromopyrazine (0.5 mmol, 0.5 eq.) were added to the reaction mixture. This mixture was stirred at 80 °C for further 16 h. Subsequently, the mixture was cooled to room temperature and 0.1 g sodium hydroxide (2.5 mmol, 2.5 eq.) was added and stirred again for 3 h at 100 °C. Adding sodium hydroxide is only needed in the case of tosyl protected indoles. The mixture was allowed to cool down to room temperature diluted with DCM and adsorbed on Celite® under reduced pressure. The adsorbed residue was purified by column chromatography on silica gel. The resulting solid was then dried for 16 h under reduced pressure (3 · 10⁻³ mbar) and a yellow solid (0.191 g, 50%) was obtained.

### Example 2

### Synthesis of the compound according to formula (3): 2,5-bis(5-bromo-1H-indole-3-yl)pyrazine

The compound 2,5-bis(5-bromo-*1H*-indole-3-yl)pyrazine was synthesised by the procedure as described in example 1, differing in that 5-bromo-3-iodo-1-tosyl-indole was used instead of 5-chloro-3-iodo-1-tosyl-indole as starting compound 1. 2,5-bis(5-bromo-*1H-*indole-3-yl)pyrazine was obtained as a yellow solid.

### Example 3

### Synthesis of the compound according to formula (4): 3,3'-(pyrimidine-2,5-diyl)bis(5-chloro-1H-indole)

The compound 3,3'-(pyrimidine-2,5-diyl)bis(5-chloro-1H-indole) was synthesised by the procedure as described in example 1, differing in that 2,5-dibromopyrimidine instead of 2,5-dibromopyrazine was added as starting compound 2 to the reaction mixture. 3,3'-(pyrimidine-2,5-diyl)bis(5-chloro-1*H*-indole) was obtained as a yellow solid.

### Example 4

### Synthesis of the compound according to formula (18): 2,4-di(1H-indole-3-yl)thiazole

The compound 2,4-di(1*H*-indole-3-yl)thiazole was synthesised by the procedure as described in example 1, differing in that 3-iodo-1-tosyl-indole and ,5-dibromo-1,3-thiazole were used as starting compounds 1 and 2, respectively. 2,4-di(1*H*-indole-3-yl)thiazole was obtained as a green-yellow solid.

### Comparative examples 5 to 10

### Synthesis of the compounds according to formulas (5) to (10)

The compounds according to formulas (5) to (10) were synthesised by the procedure as described in example 1, differing in that the starting compounds as summarised in table 1 were used. The compounds according to formulas (5) to (10) were obtained as yellow solids.

**Table 1: Alocasin A derivatives for comparison**

| Formula | | compound | starting compound 1 | starting compound 2 |
|---|---|---|---|---|
| (5) | 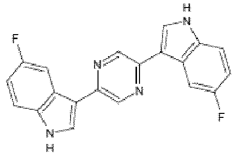 | 2,5-bis(5-fluoro-*1H-*indole-3-yl)pyrazine | 5-fluoro-3-iodo-1-tosyl-indole | 2,5-dibromo-pyrazine |
| (6) | 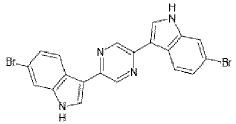 | 2,5-bis(6-bromo-*1H-*indole-3-yl)pyrazine | 6-bromo-3-iodo-1-tosyl-indole | 2,5-dibromo-pyrazine |
| (7) | 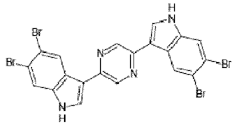 | 2,5-bis(5,6-dibromo-*1H*-indole-3-yl)pyrazine | 5,6-dibromo-3-iodo-1-tosyl-indole | 2,5-dibromo-pyrazine |
| (8) | 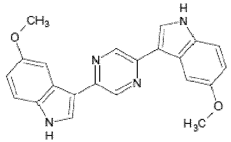 | 2,5-bis(5-methoxy-*1H*-indole-3-yl)pyrazine | 5-methoxy-3-iodo-1-tosyl-indole | 2,5-dibromo-pyrazine |
| (9) | 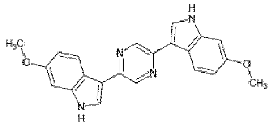 | 2,5-bis(6-methoxy-*1H*-indole-3-yl)pyrazine | 6-methoxy-3-iodo-1-tosyl-indole | 2,5-dibromo-pyrazine |
| (10) | 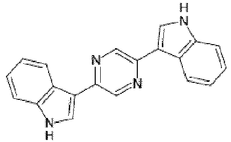 | 2,5-bis(*1H*-indole-3-yl)pyrazine | 3-iodo-1-tosyl-indole | 2,5-dibromo-pyrazine |

### Comparative examples 11 to 17

### Synthesis of the compounds according to formulas (11) to (17)

The compounds according to formulas (11) to (17) were synthesised by the procedure as described in example 3, differing in that the starting compounds as summarised in table 2 were used. The compounds according to formulas (11) to (17) were obtained as yellow solids.

**Table 2: Hyrtinadine A derivatives for comparison**

| Formula | | compound | starting compound 1 | starting compound 2 |
|---|---|---|---|---|
| (11) | 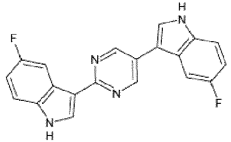 | 3,3'-(pyrimidine-2,5-diyl)bis(5-fluoro-1*H*-indole) | 5-fluoro-3-iodo-1-tosyl-indole | 2,5-dibromo-pyrimidine |
| (12) | 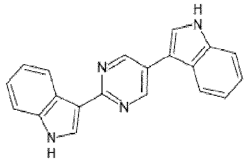 | 3,3'-(pyrimidine-2,5-diyl)bis(1*H*-indole) | 3-iodo-1-tosyl-indole | 2,5-dibromo-pyrimidine |
| (13) | 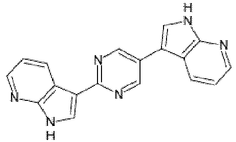 | 3,3'-(pyrimidine-2,5-diyl)bis(1*H-*pyrrolo[2,3-*b*]pyridine) | 3-iodo-1-tosyl-1*H-*pyrrolo[2,3-*b*]pyridine | 2,5-dibromo-pyrimidine |
| (14) | 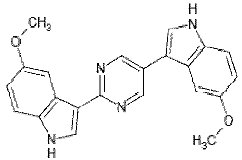 | 3,3'-(pyrimidine-2,5-diyl)bis(5-methoxy-1*H*-indole) | 5-methoxy-3-iodo-1-tosyl-indole | 2,5-dibromo-pyrimidine |
| (15) | 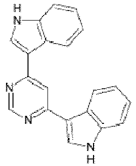 | 4,6-di(1*H*-indole-3-yl)pyrimidine | 3-iodo-1-tosyl-indole | 4,6-dibromo-pyrimidine |
| (16) | 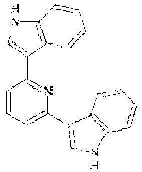 | 2,6-di(1*H*-indole-3-yl)pyridine | 3-iodo-1-tosyl-indole | 2,6-dibromo-pyridine |
| (17) | 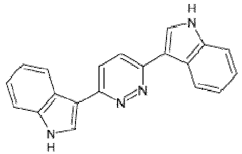 | 3,6-Di(1*H*-indole-3-yl)pyridazine | 3-iodo-1-tosyl-indole | 3,6-dibromo-pyridazine |

### Example 18

### Determination of minimal inhibitory concentration (MIC) against nosocomial pathogens and Micobacterium tuberculosis

### A) Determination by the broth microdilution method

The compounds were tested for their antibacterial activities against the pathogenic nosocomial strains of *Staphylococcus aureus, Enterococcus faecalis Enterococcus faecium, Acinetobacter baumannii.* The minimal inhibitory concentration (MIC) was determined by the broth microdilution method according to the recommendations of the Clinical and Laboratory Standards Institute (CLSI) (CLSI M7-A9, 2012). For preparation of the inoculum the growth method was used. Bacterial cells were grown aerobically in Muller Hinton (MH) medium at 37°C and 180 rpm. A preculture was grown until log phase (OD_{600 nm} ∼ 0.5) and then seeded at 5 x 10⁴ bacteria/well in a total volume of 100 µL in 96 well round bottom microtiter plates and incubated with serially diluted test substances at a concentration range of 100-0.78 µM. The compounds were screened in two-fold serial dilution. Microplates were incubated aerobically at 37°C for 24 h. MICs were determined by identifying the minimum concentration of the compounds that led to complete inhibition of visual growth of the bacteria.

For the determination of MIC against *M. tuberculosis,* 1x10⁵ bacteria/well were seeded in a total volume of 100 µL in 96 well round bottom microtiter plates and incubated with serially diluted test compounds at a concentration range of 100-0.78 µM. Again, the compounds were screened in two-fold serial dilution. Microplates were incubated at 37°C for five days. Afterwards, 10 µL/well of a 100 µg/mL resazurin solution were added and incubated at ambient temperature for further 16 h. Then cells were fixed for 30 min after formalin addition (5%, v/v, final concentration). For viability determination, fluorescence was quantified using a microplate reader (excitation 540 nm, emission 590 nm). Percentage of growth was calculated relative to rifampicin treated (0% growth) and DMSO treated (100% growth) controls.

**The Table 3: results of the minimal inhibitory concentration (MIC) against the nosocomial pathogens and Mycobacterium tuberculosis**

| Compound | MIC [µM] | | | | | | |
|---|---|---|---|---|---|---|---|
| formula | *M*. *tuberculos is* H37Rv | *S. aureus* ATCC 25923 | MRSA ATCC 700699 | *E. faecalis* ATCC 29212 | *E. faecalis* ATCC 51299 | *E. faecium* ATCC 35667 | VR *E*. *faecium* ATCC 700221 |
| (2) | > 100 | > 100 | 0.39 | > 100 | > 100 | > 100 | > 100 |
| (3) | > 100 | 1.56 | 1.56 | > 100 | > 100 | > 100 | > 100 |
| (4) | 50 | 6.25 | 0.39 | 3.125 | 12.5 | 1.56 | 1.56 |
| (5) | > 100 | > 100 | > 100 | > 100 | > 100 | > 100 | > 100 |
| (6) | > 100 | > 100 | > 100 | > 100 | > 100 | > 100 | > 100 |
| (7) | > 100 | 100 | 25 | 100 | > 100 | 50 | 50 |
| (8) | > 100 | > 100 | > 100 | > 100 | > 100 | > 100 | > 100 |
| (9) | > 100 | > 100 | > 100 | > 100 | > 100 | > 100 | > 100 |
| (10) | > 100 | > 100 | > 100 | 12.5 | 25 | > 100 | > 100 |
| (11) | > 100 | 100 | 12.5 | 100 | 100 | 50 | 50 |
| (12) | 50 | 100 | 12.5 | 50 | 100 | 100 | 100 |
| (13) | > 100 | > 100 | > 100 | 100 | > 100 | > 100 | > 100 |
| (14) | 50 | > 100 | 50 | 50 | 100 | 100 | 50 |
| (15) | > 100 | > 100 | > 100 | > 100 | > 100 | > 100 | > 100 |
| (16) | > 100 | > 100 | > 100 | 100 | 100 | > 100 | > 100 |
| (17) | > 100 | > 100 | > 100 | 100 | 100 | > 100 | > 100 |
| (18) | 100 | 6.25 | 3.125 | > 100 | > 100 | 6.25 | 6.25 |

As can be taken from table 3, the compounds showed different inhibitory activity against the tested Gram-positive bacterial strains. The MRSA strain was effectively inhibited by the alocasin derivatives according to formulas (2) and (3). The compound according to formula (2) inhibited exclusively MRSA (MIC = 0.39 µM), while the compound according to formula (3) showed a MIC of 1.56 µM against the methicillin-sensitive parental *S. aureus* strain as well.

The methoxylated and unsubstituted alocasin derivatives of formulas (8), (9) and (10) did not show any effect against *M. tuberculosis, S. aureus* and *E.faecium,* and the unsubstituted alocasin derivative had only moderate activity against both *E.faecalis* strains. When the compound was halogenated at position 5,5' of the indole ring, the inhibitory concentration decreased. The fluorinated compound according to formula (5) had no activity against the tested strains. Paradoxically, a decreased inhibitory effect for compound according to formula (2) could be observed at concentrations ≥ 3.125 µM. It is assumed that the compound according to formula (2) aggregates at higher concentrations because of its axial symmetry and precipitates, thus resulting in a decreased concentration of the compound in solution. The additional halogenation at position 6,6' of the indole rings led to an increased MIC up to 25-100 µM. If only position 6,6' was halogenated like in compound according to formula (6), no activity could be observed in the tested concentration range.

Regarding the halogenated hyrtinadine derivatives according to formulas (4) and (11), it could also be observed that the replacement of chlorine with fluorine led to a decrease of the MIC with factors between 8 and 32. The methoxylated and unsubstituted hyrtinadine derivatives had only a slight effect against *M. tuberculosis*, *S. aureus* and *E. faecium.*

These results indicate that the observed antibacterial potencies were highly depending on the different moieties at position 5 and 5' or 6 and 6' of the indole rings. A halogenation with chlorine or bromine seems to be essential for the antimicrobial activity. However, none of the tested compounds were active against the Gram-negative bacterium *Acinetobacter baumannii.*

Regarding the compound of formula (18) having a 5-membered thiazole ring as linker A, the compound inhibited the growth *of S. aureus* and *E. faecium* strains with an MIC of 6.25 µM.

### B) Determination via the BacTiter-Glo™ assay

The MICs of compounds according to formulas (2), (3), (4), (7), (11), (12) and (18) were additionally quantified via the BacTiter-Glo™ assay (Promega, Madison, WI, USA). Briefly, after an incubation time of 24 h, each well was resuspended by pipetting, and 50 µL aliquots were transferred into an opaque-walled multiwell plate. Additionally, control wells containing medium without cells to determine background luminescence were prepared. 50 µL BacTiter-Glo™ reagent were added to each well. Contents were mixed and incubated for five minutes at room temperature. Luminescence was measured using a microplate reader. Growth was calculated relative to the background luminescence (0% growth) and a compound-free DMSO control (100% growth).

The results confirmed that the chlorinated and brominated compounds according to formulas (2), (3) and (4) as well as the compound according to formula (18) effectively inhibited the methicillin-resistant and -sensitive *Staphylococcus aureus* strains.

### Example 19

### Determination of cytotoxicity and therapeutic index

The cytotoxicity of the compounds according to formulas (2), (3) and (4) and according to formulas (7), (11), (12) and (18) was determined *in vitro* using the human monocyte cell line THP-1 (Deutsche Sammlung von Mikroorganismen und Zellkulturen GmbH) and the human fetal lung fibroblast cell line MRC-5 (American Type Culture Collection).

THP-1 cells were cultured in RPMI 1640 medium containing 10% (v/v) fetal bovine serum (FBS), while MRC-5 cells were incubated in Dulbecco's Modified Eagles Medium (DMEM) containing 10% (v/v) FBS both at 37°C in a humidified atmosphere of 5% CO₂. Cells were seeded at approx. 5*10⁴ cells/well in a total volume of 100 µL in 96 well flat bottom microtiter plates containing twofold serially diluted test compounds at a maximum final concentration of 100 µM. Cells treated with DMSO in a final concentration of 1% (v/v) served as DMSO toxicity controls. After an incubation time of 48 h, 10 µL resazurin solution (100 µg/mL) were added per well and incubated for further 3 h at 37°C in a humidified atmosphere of 5% CO₂. Fluorescence was quantified using a microplate reader (excitation 540 nm, emission 590 nm). Growth was calculated relative to non-inoculated (i.e. cell-free) (0% growth) and untreated (100% growth) controls in triplicate experiments, respectively. For determination of the therapeutic index of the tested substances, the selectivity index (SI) was determined by the quotient of cytotoxic concentration and MIC. Compounds with SI ≥ 10 were characterized in further experiments.

The Figure 1 shows the dose-response curves of the compound according to formula (2) in Figure 1a), the compound according to formula (3) in Figure 1b), the compound according to formula (4) in Figure 1c), and the compound according to formula (18) in Figure 1d) against MRSA and the human cell lines MRC-5 and THP-1. As can be seen in Figures 1a) and b), all compounds were cytotoxic against THP-1 and MRC-5 cells only at an inhibitory concentration of 12.5 µM for the compound according to formulas (2) and (3). Since compounds according to formulas (2), (3) and (4) are cytotoxic at concentrations >12.5 µM, the selectivity indices of these substances are favorable, yielding sufficient therapeutic margins.

### Example 20

### Determination of time-kill curves in vitro

A killing kinetic was performed to determine the potential bactericidal or bacteriostatic effect of the tested compounds on actively growing bacteria. Killing kinetics were measured for the compounds according to formulas (2), (3) and (4) against the *Staphylococcus aureus* MRSA strain ATCC 700699. An overnight culture of the strain was adjusted to a final inoculum concentration of 10⁶ CFU/mL cultivated in MH broth containing the tested compounds at five-fold MIC, for the compounds according to formulas (2) and (4) at a concentration 2 µM, and for compound according to formula (3) at 8 µM, respectively, resulting in 0.02-0.08% DMSO final concentration. Cultures containing moxifloxacin at five-fold MIC (20 µM) were used as positive control. Furthermore, MRSA cultures cultivated in MH broth with 0.02% DMSO, correlating to the maximum amount of DMSO in the compound containing cultures, were handled as antibiotic-free control.

In a second attempt, the culture medium was removed after 6 h post exposure to compounds and replaced by fresh compound-containing medium. This should overcome limited antibacterial activity in case a compound was degraded or consumed during the experiment. Therefore, the culture was centrifuged at 4000 rpm for 10 min. Afterwards, the bacterial cell pellet was washed in PBS and resuspended in fresh medium containing the corresponding compound at the initial concentration.

Shaking cultures were incubated for 24 h at 37°C at 180 rpm. After 0, 3, 6 and 24 h of incubation, viability, expressed as CFU/mL, was determined by plating 100 µL aliquots of a serial dilution onto MH agar plates. The plates were incubated aerobically at 37°C for 24. All killing kinetics were performed in duplicates. The results are presented as means of the duplicates ±SD.

Additionally, the resistance rate of the cultures was determined after 6 and 24 h of incubation. For this, 20 µL of the cultures were plated on 1 mL MH agar containing the five-fold MIC of the compounds (for the compound according to formula (2) and (4): 2 µM, for the compound according to formula (3): 8 µM). After an incubation time of 24 h at 37°C, the colonies were counted. The resistance rate was determined as quotient of the number of resistant colonies on compound containing agar and the total amount of viable bacteria at the corresponding time points.

The Figure 2 shows the time-kill curves of methicillin resistant *S. aureus* (MRSA; ATCC 700699) for the compound according to formula (2) in Figure 2a), the compound according to formula (3) in Figure 2b), and the compound according to formula (4) in Figure 2c) against Moxifloxacin positive and antibiotic-free negative controls, respectively. Also included are the results of the independent experiment, where medium was replaced and compounds were freshly added after 6 h.

As can be taken from the Figure 2, it could be shown that the compounds according to formulas (2), (3) and (4) had a strong bactericidal killing effect. After an incubation time of 6 h the bacteria amount was reduced to 10² and <10¹ CFU/mL by the compounds according to formulas (3) and (4), respectively, at the five-fold MIC. The treatment with compound of formula (2) led to a reduction from 10⁶ to 10⁴ CFU/mL. Since after 24 h the amount of surviving bacteria remained <10¹ CFU/mL (representing the limit of detection) by the compound of formula (4) containing medium, this highly bactericidal substance is able to sterilize the culture. In contrast, some persistent bacteria remained in the compounds of formulas (2) and (3) containing cultures after 6 h of treatment, giving rise to increasing number of cells again at prolonged treatment intervals.

When compound according to formula (2) was freshly added to washed cells after 6 h of incubation, the viability of the culture continued to decrease to 10² CFU/mL initially, but almost rose to the same amount than the growth control after 24 h incubation time. Accordingly, the compound according to formula (2) seems to be rapidly degraded or depleted in liquid culture such that antibacterial effect wanes after more than 6 h incubation time. Regrowth of the pathogen at extended incubation times was much less pronounced when compound according to formula (3) was freshly added to the culture after 6 h. it is therefore assumed that substance according to formula (3) might be less prone to degradation or depletion than structure according to formula (2).

The Figure 3 shows the resistance development in Methicillin resistant *S. aureus* (MRSA; ATCC 700699) during time-kill kinetics of the compounds of formulas (2) and (3) in the Figures 3a) and 3b), respectively. Two independent cultures C1 and C2 were tested for each attempt. Compounds were tested at 5x MIC resulting in 0.02-0.08% DMSO final concentration. In an independent experiment, medium was replaced and compounds were freshly added after 6 h, denoted "freshly added" in the Figure. 20 µL of different dilution of the cultures after either 6 or 24 h of incubation were plated out on MH agar containing either no antibiotics, which is denoted (*) or the compounds of formulas (2) and (3) at 5x MIC, respectively. As can be seen in Figure 3, no significant level of resistance was detectable in these treated cultures after 6 and 24 h of incubation with the compounds of formulas (2) and (3).

### Example 21

### Determination of the mode of resistance

### a) Determination of level of resistance

In order to gain insight into the potential mode-of-action and the molecular target(s), spontaneous MRSA mutants were isolated on solid medium containing the compounds of formulas (4) or (2) at 3- or 5-fold MIC. For this, 20 µL of bacterial cell suspensions of 1×10⁶, 1×10⁷ and 1×10⁸ CFU/mL of the methicillin-resistant *S. aureus* (MRSA) strain ATCC 700699 were spread on MH agar plates containing the compounds of formulas (2) or (4) at 3- and 5-fold MIC. After 72 h of incubation at 37°C, resistant colonies were quantified. Spontaneous mutants resistant to compound of formula (2) developed at a frequency of 5×10⁻⁷, whereas spontaneous mutants resistant to compound of formula (4) occurred at a frequency of 5×10⁻⁵. The isolated individual resistant mutants were subsequently tested against the compound according to formula (4) to determine their level of resistance.

Figure 4 shows the dose-response curves of resistant MRSA mutants for the compound according to formula (4). Mutants were generated on agar containing 3-fold MIC of the compounds of formulas (4) or (2), denoted 9-RM or 1-RM, respectively, or with 5-fold MIC of the compounds of formulas (4), denoted 9-RM-5x. Parent MRSA strain (MRSA WT) was used as control. Figure 4 shows that the MIC of the compounds of formulas (4) was 8-fold higher for the mutants than for the parent MRSA strain. Also the two tested clones that were resistant against compound according to formula (2) showed cross-resistance against compound according to formula (4), indicative of a common mode-of-action and target of these compounds.

### b) Isolation of genomic DNA and whole genome sequencing

For isolation of the genomic DNA, individual resistant mutants were cultivated overnight. Bacteria suspensions were centrifuged at 4000 rpm for 5 min. Afterwards, bacterial cell pellets were suspended in a lysostaphin-GTE solution (200 µg/mL lysostaphin in 25 mM Tris-Cl, pH 8.0; 10 mM EDTA; 50 mM glucose) and incubated at 37°C for 30 min. After addition of 5 µL proteinase K (10 mg/mL), the suspensions were incubated at 56°C for 1 h. Subsequently, an equal volume of 24:1 chloroform/isoamyl alcohol was added. The mixture was shaken vigorously and spun at 4000 rpm for 10 min. The aqueous layer was transferred in a fresh tube and the extraction step was repeated. DNA was precipitated by addition of 60 µL NaCl (5 M) and 1 mL ethanol. After centrifugation and aspiration of the supernatant, the DNA pellet was air-dried for 15 min. Finally, the DNA was solved in TE buffer containing RNAse (10 mM Tris, 0,1 mM EDTA, pH 7.4).

Whole-genome resequencing of four individual mutants resistant against the compound according to formula (4) and of two mutants resistant against the compound according to formula (2) was performed to reveal the resistance-mediating determinants, some of which might directly be linked to the molecular target. Libraries were prepared for sequencing using the standard paired-end genomic DNA sample prep kit from Illumina. Genomes were sequenced using an Illumina HiSeq 2500 next-generation sequencer (San Diego, CA, USA) and compared with the parent methicillin resistant *S. aureus* genome (strain ATCC 700699 (= Mu50); GenBank accession GCA_000009665.1). Paired-end sequence data was collected with a read length of 106 bp. Base-calling was performed using Casava software, v1.8. The reads were assembled using a comparative genome assembly method. The mean depth of coverage ranged from 277× - 770x.

It was found that all six clones harbored a single nucleotide polymorphisms (SNP) in the gene SAV_RS01095: Mutants 9-RM3, 1-RM1 and 1-RM3 carried a mutation that causes a substitution of the amino acid at position 674 from glycine to aspartic acid. Furthermore, mutants 9-RM2-5x, 9-RM3-5x, 9-RM5-5x had a SNP that leads to an amino acid substitution at position 461 from valine to leucine. The common mutations in gene SAV_RS01095 in six individual mutants strongly suggests that this gene plays a direct role in resistance, and the corresponding protein might possibly even represent a direct cellular target for the compounds according to formulas (4) and (2). However, the molecular consequences of the amino acid substitutions for function of the protein as well as the molecular principle underlying resistance are currently unknown. The gene SAV_RS01095 encodes the enzyme EIICBA from the phosphoenolpyruvate-dependent glucose phosphotransferase system (PTS). The PTS catalyzes the phosphorylation of incoming sugar substrates associated with their translocation through the cell membrane. It is thus assumed that the compounds according to formulas (4) and (2) might inhibit growth *of S. aureus* by blocking glucose utilization via targeting the enzyme EIICBA.

In summary, the results show a strong bactericidal effect of the compounds according to formulas (2), (3), (4) and (18), particularly against methicillin-resistant *Staphylococcus aureus* (MRSA)

## Claims

1. A compound according to general formula (1) as given as follows and/or racemates, enantiomers, stereoisomers, solvates, hydrates, and pharmaceutically acceptable salts and/or esters thereof: 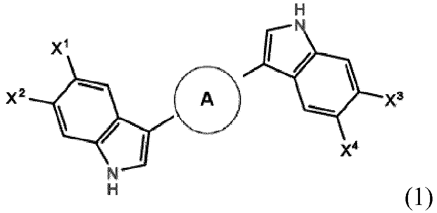 wherein:
A is a 6-membered heteroaromatic ring selected from the group consisting of pyridyl, pyridazyl, pyrimidyl and/or pyrazyl, or a 5-membered heteroaromatic ring selected from the group consisting of thiazolyl, oxazolyl, imidazolyl, pyrazolyl, thiophenyl, furyl and/or pyrrolyl; and
X¹, X⁴ are selected from Cl or Br and X², X³ are H if A is a 6-membered heteroaromatic ring, or
X¹, X², X³, X⁴ are the same or independent from each other selected from the group consisting of H, Cl and Br if A is a 5-membered heteroaromatic ring,
for use in the treatment of a bacterial infection.

2. The compound for use according to claim 1, wherein A is pyrimidyl or pyrazyl, X¹, X⁴ are selected from Cl or Br, and X², X³ are hydrogen.

3. The compound for use according to claim 1, wherein A is thiazolyl and X¹, X², X³ and X⁴ are hydrogen.

4. The compound for use according to claims 1 or 2, wherein the compound is selected from the group of compounds according to formulas (2) to (4) as given as follows:

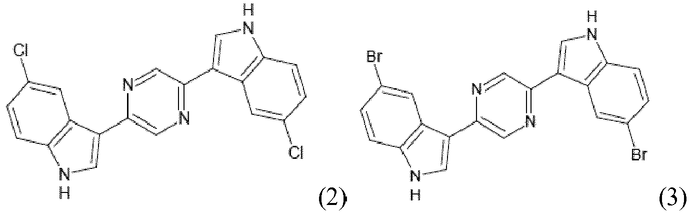

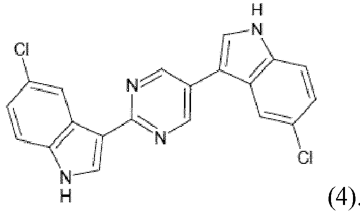

5. The compound for use according to claims 1 to 4, wherein the bacterial infection is an infection with *Staphylococcus aureus,* particularly with methicillin resistant *Staphylococcus aureus* (MRSA).

6. A pharmaceutical composition comprising as an active ingredient a compound according to general formula (1) and/or racemates, enantiomers, stereoisomers, solvates, hydrates, and pharmaceutically acceptable salts and/or esters thereof: 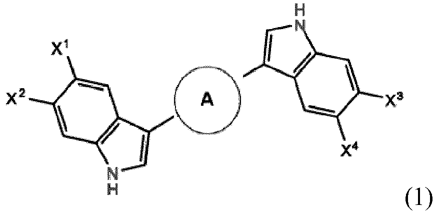 wherein:
A is a 6-membered heteroaromatic ring selected from the group consisting of pyridyl, pyridazyl, pyrimidyl and/or pyrazyl, or a 5-membered heteroaromatic ring selected from the group consisting of thiazolyl, oxazolyl, imidazolyl, pyrazolyl, thiophenyl, furyl and/or pyrrolyl; and
X¹, X⁴ are selected from Cl or Br and X², X³ are H if A is a 6-membered heteroaromatic ring, or
X¹, X², X³, X⁴ are the same or independent from each other selected from the group consisting of H, Cl and Br if A is a 5-membered heteroaromatic ring,
for use in the treatment of a bacterial infection.

7. The pharmaceutical composition for use according to claim 6, wherein the composition comprises a liquid or solid carrier.

8. The pharmaceutical composition for use according to claim 6, wherein the bacterial infection is an infection with *Staphylococcus aureus,* particularly with methicillin resistant *Staphylococcus aureus* (MRSA).

9. Use of a compound according to general formula (1) and/or racemates, enantiomers, stereoisomers, solvates, hydrates, and pharmaceutically acceptable salts and/or esters thereof: 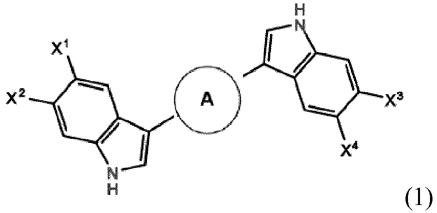 wherein:
A is a 6-membered heteroaromatic ring selected from the group consisting of pyridyl, pyridazyl, pyrimidyl and/or pyrazyl, or a 5-membered heteroaromatic ring selected from the group consisting of thiazolyl, oxazolyl, imidazolyl, pyrazolyl, thiophenyl, furyl and/or pyrrolyl; and
X¹, X⁴ are selected from Cl or Br and X², X³ are hydrogen if A is a 6-membered heteroaromatic ring, or
X¹, X², X³, X⁴ are the same or independent from each other selected from the group consisting of H, Cl and Br if A is a 5-membered heteroaromatic ring,
for the manufacture of a medicament for the treatment of a bacterial infection.

10. The use of claim 8, wherein the bacterial infection is an infection with *Staphylococcus aureus,* particularly with methicillin resistant *Staphylococcus aureus* (MRSA).

11. A method of treating a bacterial infection, the method comprising the step of administering to a subject a therapeutically effective amount of a compound according to general formula (1) and/or racemates, enantiomers, stereoisomers, solvates, hydrates, and pharmaceutically acceptable salts and/or esters thereof: 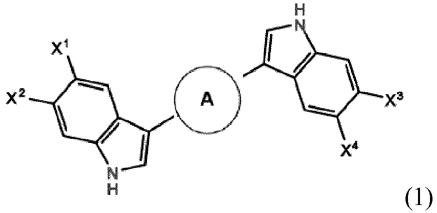 wherein:
A is a 6-membered heteroaromatic ring selected from the group consisting of pyridyl, pyridazyl, pyrimidyl and/or pyrazyl, or a 5-membered heteroaromatic ring selected from the group consisting of thiazolyl, oxazolyl, imidazolyl, pyrazolyl, thiophenyl, furyl and/or pyrrolyl; and
X¹, X⁴ are selected from Cl or Br and X², X³ are hydrogen if A is a 6-membered heteroaromatic ring, or
X¹, X², X³, X⁴ are the same or independent from each other selected from the group consisting of H, Cl and Br if A is a 5-membered heteroaromatic ring.

12. The method according to claim 10, wherein the bacterial infection is an infection with *Staphylococcus aureus,* particularly with methicillin resistant *Staphylococcus aureus* (MRSA).

13. A compound selected from the group of compounds according to formulas (2), (5), (7) and (9) as given as follows:

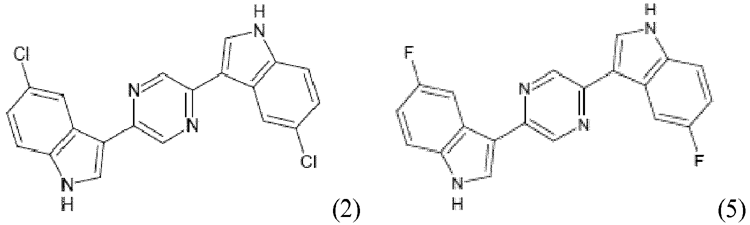

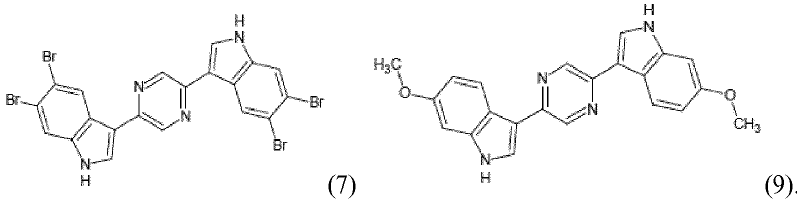

14. The compounds according to formulas (2) and (18) for use as a medicament.

15. An inhibitor of the enzyme EIICBA, particularly an inhibitor according to general formula (1) as given as follows and/or racemates, enantiomers, stereoisomers, solvates, hydrates, and pharmaceutically acceptable salts and/or esters thereof: 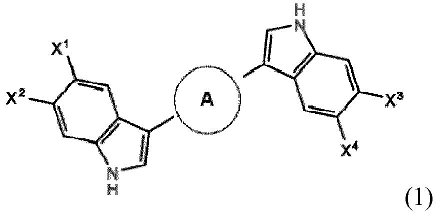 wherein:
A is a 6-membered heteroaromatic ring selected from the group consisting of pyridyl, pyridazyl, pyrimidyl and/or pyrazyl, or a 5-membered heteroaromatic ring selected from the group consisting of thiazolyl, oxazolyl, imidazolyl, pyrazolyl, thiophenyl, furyl and/or pyrrolyl; and
X¹, X⁴ are selected from Cl or Br and X², X³ are hydrogen if A is a 6-membered heteroaromatic ring, or
X¹ X², X³, X⁴ are the same or independent from each other selected from the group consisting of H, Cl and Br if A is a 5-membered heteroaromatic ring.
